# EUROPEAN PATENT APPLICATION

(11) **EP 2 275 149 A2**
(43) Date of publication of application: **19.01.2011**
(21) Application number: 10251274.6
(22) Date of filing: 16.07.2010
(51) Int. Cl.: A61L 17/10

(54) **Heterogeneous yarns for surgical articles**

(30) Priority: 17.07.2009 US 504954
(71) Applicant: Tyco Healthcare Group LP, New Haven, CT 06511 (US)
(72) Inventor: Kennedy, John J., Guilford, CT 06437 (US); Stopek, Joshua, Guilford, CT 06437 (US); Roby, Mark S., Killingworth, CT 06419 (US)
(74) Representative: Soames, Candida Jane

(57) **Abstract**

Heterogeneous yarns containing strands of dissimilar materials are useful in forming surgical device.

## Description

### CROSS-REFERENCE TO RELATED APPLICATIONS

The present application is a continuation-in-part of, and claims the benefit of and priority to, co-pending United States Patent Application Serial No. 10/917,183 filed on August 12, 2004, which claims priority to and the benefit of United States Provisional Application No. 60/494,993 filed on August 14, 2003, the entire disclosures of each of which are hereby incorporated by reference in their entirety.

### BACKGROUND

### 1. Technical Field

The present disclosure relates to yarns made of dissimilar materials. Filaments of at least two dissimilar materials are combined to form a yarn which, in turn, can be braided, woven, etc. to form a device suitable for surgical use.

### 2. Background of Related Art

Sutures intended for the repair of body tissues must meet certain requirements; they must be substantially non-toxic, capable of being readily sterilized, they must have good tensile strength and have acceptable knot-tying and knot-holding characteristics and, if the sutures are of the bio-absorbable variety, the bio-absorption of the suture must be closely controlled.

Sutures have been constructed from a wide variety of materials including surgical gut, silk, cotton, polyolefins such as polypropylene, polyamides, polyesters such as polyethylene terephthalate, polyglycolic acid, glycolide-lactide copolymer, etc. Sutures have been constructed from these materials in a monofilament form and as braided structures. For example, sutures manufactured from silk, polyamide, polyester and bio-absorbable glycolide-lactide copolymer are typically provided as multi-filament braids.

Filaments have also been utilized to form other mutli-filament surgical or medical devices, such as braided tapes, gauze, wound dressings, hernial repair meshes, vascular grafts (e.g. fabrics and/or tubes) anastomosis rings, prosthetic ligaments and tendons, growth matrices, drug delivery devices and other implantable medical devices. Multi-filaments braids typically provide the advantages of enhanced pliability, and tensile strength as compared to monofilament constructions, and where utilized as sutures, posses enhanced knot security. The enhanced pliability of a multi-filament braid is caused by a lower resistance to bending of a strand of very fine filaments as opposed to one large diameter monofilament. The individual filaments must be able to bend unencumbered or unrestricted by their neighboring filaments. Any mechanism which reduces this individual fiber mobility, such as simple fiber-fiber friction, a coating which penetrates into the braid interstices, or a melted polymer matrix which adheres fibers together, could adversely affect braid pliability. It would be advantageous to combine dissimilar materials or fibers to form filament strands to enhance pliability and strength.

### SUMMARY

The present disclosure relates to biocompatible composite surgical devices made from heterogeneous yarns. The yarns contain multiple strands of a polyester material and multiple strands of a polyolefin material. The strands are combined in substantially parallel lengths with respect to each other to form the heterogeneous yarn. The yarns can then be braided, knitted or woven to form medical/surgical devices, including sutures.

### BRIEF DESCRIPTION OF DRAWINGS

FIG. 1 is a cross sectional view of a heterogeneous yarn of polyester and polyolefin strands;

FIG. 2 shows a needle-suture combination in accordance with the present disclosure;

FIG. 3 is a perspective view of a portion of a split human sternum illustrating one application of the present invention for retaining the split portions together to promote healing;

FIG. 4 is an enlarged view of the suture product shown in FIG. 3 illustrating one embodiment wherein the elongated product is a flat braided member and contains at least eight reinforcing filaments extending along the length;

FIG. 5 is a view of an alternative embodiment of the suture repair product wherein the elongated member is a spiroid braided member having a generally circular cross-section containing at least one elongated reinforcing member; and

FIG. 6 is a view of another alternative embodiment of the suture repair product wherein the elongated product is a hollow braided member having a generally circular cross-section and contains at least one elongated reinforcing member extending centrally thereof along the length.

### DETAILED DESCRIPTION

The present disclosure relates to heterogeneous yarns, which can be used in the fabrication in whole or in part of a variety of textile surgical devices, including, for example, sutures, braided tapes, gauze, wound dressings, hernial repair meshes, vascular grafts (e.g. fabrics and/or tubes) anastomosis rings, prosthetic ligaments and tendons, growth matrices, drug delivery devices and other implantable medical devices. An implantable medical device is defined as any device which can be implanted in an animal for medical purposes. The yarns can be braided, knitted or woven to form the devices.

In accordance with the present disclosure, at least two different kinds of fibers or strands are placed in intimate contact to form a heterogeneous yarn. More particularly, the heterogeneous yarns contain strands made from a polyester and strands made from a polyolefin. Multiple strands of polyester and polyolefin are combined to form a single yarn. Multiple heterogeneous yarns thus obtained are then braided, knitted, or woven to form a multi-filament surgical/medical device, such as, for example, a multi-filament surgical suture.

In particularly useful embodiments, the polyester strands are made of polyethylene terephthalate. Polyethylene terephthalate is a thermoplastic polyester formed by esterification from ethylene glycol and terephthalic acid. Its advantageous properties include high tensile strength, high resistance to stretching under both wet and dry conditions, and good resistance to degradation by chemical bleaches and to abrasion. Polyethylene terephthalate is commercially available from DuPont Corporation, Wilmington, Del., under the trademark DACRON^{®}.

The polyolefin strands are preferably made from a polyethylene. In particularly useful embodiments, the polyethylene is an ultra high molecular weight polyethylene. Ultra high molecular weight ("UHMW") polyethylene is a linear polymer with an average molecular weight greater than about 400,000, typically in the range of about 500,000 to about 6,000,000. UHMW polyethylene has a high tenacity and low elongation rate to provide articles with greatly increased strength and decreased elongation.

UHMW polyethylene typically exhibits a very substantial degree of crystalline orientation (95-99%) and crystalline content (60-85%). The significant strength and stability of UHMW polyethylene is normally caused by the high degree of molecular orientation. As a result, the fibers exhibit strengths from about 375 kpsi (thousands of pounds per square inch) to about 560 kpsi, and tensile moduli of about 15 msi (millions of pounds per square inch) to about 30 msi. Ultra high molecular weight polyethylene is commercially available under the trademark SPECTRA^{®}. from Allied-Signal Technologies, Petersburg, Va., and under the trademark DYNEEMA^{®} from DSM High Performance Fibers, JH Heerlen, The Netherlands.

The yarn may optionally contain strands of other materials. Materials used to construct these optional strands can include a wide variety of natural and synthetic fibrous materials such as any of those previously known for the construction of sutures. Such materials include non-absorbable as well as partially and fully bio-absorbable (i.e., resorbable) natural and synthetic fiber-forming polymers, including thermoplastics. Suitable non-absorbable materials can include, for example, polyamides, polyesters such as polyethylene terephthalate, polyacrylonitrile, polyolefins such as polyethylene and polypropylene, silk, cotton, linen, etc. Carbon fibers, steel fibers and other biologically acceptable inorganic fibrous materials can also be employed. Bio-absorbable resins can include those derived from glycolic acid, glycolide, lactic acid, lactide, dioxanone, epsilon-caprolactone, trimethylene carbonate, etc., and various combinations of these and related monomers.

The strands which form the yarns can be made using any known technique, such as, for example, extrusion, molding and/or solvent casting. In a preferred embodiment, the strands can be extruded through an extruder unit of a conventional type, such as those disclosed in U.S. Pat. Nos. 6,063,105; 6,203,564; and 6,235,869, the contents of each of which are incorporated by reference herein. The strands of dissimilar materials can be extruded separately and subsequently brought together into a group to form a yarn, or the strands can be extruded in a side-by-side fashion and collected together to immediately form a yarn. The number of strands used per yarn will depend on a number of factors including the desired final size of the yarn and the ultimate multi-filament article being produced. For example, with respect to sutures, size is established according to United States Pharmacopoeia ("USP") standards. However, each yarn can contain at least ten strands, and often more, of at least two differing material types. The strands run parallel to each other along the length of the yarn. Although twisting the strands to form a twisted yarn is also contemplated.

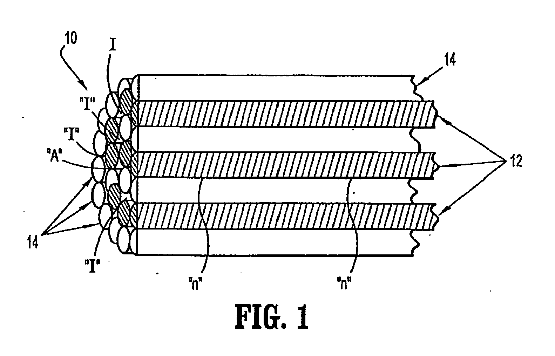

Turning now to FIG. 1, heterogeneous yarn 10 of the present disclosure is shown. Yam 10 may be formed, in embodiments extruded, to any suitable length. More particularly, the extruded length of the yarn 10 may be determined by the intent of use of the yarn 10. For example, in the instance where the intended use of the yarn 10 is for the manufacture of a suture 101, the yarn 10 includes a length that is at least equal to the length of the suture (see FIG, 2, for example). In embodiments, yarn 10 may include lengths that are from about 5 inches to about 144 inches. For illustrative purposes, a segment of a yarn 10 is depicted in FIG. 1 and includes a plurality of polyolefin strands 12 (such as, UHMW polyethylene) and polyester strands 14 (such as DACRON^{®}). While only a segment of yarn 10 is depicted in FIG. 1, it is to be understood that, in embodiments, the parallel orientation of strands 12 and 14 may be maintained along the entire length of yarn 10 (not shown). In other words, the plurality of strands 12 and 14 run parallel to both each other and the longitudinal axis of yarn 10, along the length of yarn 10.

As noted above, yarn 10 may be formed by one or more known extrusion processes. After the strands 12 and 14 have been extruded and subsequently brought together and/or collected, each of the strands 12 and 14 are arranged and maintained so that they are in intimate contact with each other in a parallel relation relative to one another. This parallel configuration of strands 12 and 14 may increase the structural integrity of the yarn 10.

The strands 12 and 14 may be maintained in this parallel relation by any suitable methods and/or processes. For example, in an embodiment, the strands 12 and 14 may be temporarily positioned within a holding receptacle, such as, for example, a removable forming sheath (not shown). The forming sheath may be configured to maintain the strands 12 and 14 in a parallel configuration during the formation of the yarn 10.

In embodiments, after the strands 12 and 14 have been positioned within the forming sheath, the strands 12 and 14 may be subsequently bonded to each other by any means within the purview of those skilled in the art. Such means may include, for example, the application of heat, the use of binding agents, the use of coatings, combinations thereof, and the like. In embodiments, instead of a forming sheath, a permanent sheath may be applied to the external surface of yarn 10, thereby assisting in maintaining the parallel orientation of strands 12 and 14.

A sheath which is applied to the strands 12 and 14 and left thereon to maintain their parallel orientation may be formed of any materials used to form strands 12 and/or 14, as well as any other component utilized to form a suture of the present disclosure.

Depending upon the materials utilized to form the strands, in embodiments, suitable heating to fuse the strands together may include heating to from about 70°C to about 160°C, in embodiments from about 100°C to about 140°C.

The heating may be dependent upon the glass transition temperature of the materials utilized to form the strands. Moreover, in embodiments, it may be desirable to heat the strands under tension, to maintain the parallel orientation of the strands.

Suitable binding agents are within the purview of those skilled in the art and include suitable biocompatible adhesives, thermoplastic resins, waxes, combinations thereof, and the like. In embodiments, a plurality of interstices "I" may be formed between the plurality of strands 12 and 14. The interstices "I" run parallel to each other and the strands 12 and 14 along the length of the yarn 10. In embodiments, the plurality of interstices may be configured to provide an area for receiving the binding agent "A". The binding agent "A" may be applied along the entire length of the yarn, or in discrete locations along the length of the yarn. In embodiments it may prove useful to apply the binding agent may be deposited at specific locations or nodes "n" along the length of the yarn, see FIG. 1. The plurality of nodes "n" and/or forming sheath may be configured to receive the binding agent at corresponding predetermined locations along the length of the yarn 10. The plurality of nodes "n" may be configured to maintain the strands 12 and 14 in a fixed parallel relation relative to one another.

Thus, in embodiments, a method for forming a yarn of the present disclosure may include positioning a plurality of strands in side-by-side fashion such that the plurality of strands are maintained in a parallel relation relative to one another, applying a binding agent to the plurality of heterogeneous strands, and setting the binding agent to assist in maintaining the strands in their parallel configuration.

Any biocompatible adhesive may be utilized. In embodiments, suitable adhesive materials include synthetic absorbable and non-absorbable monomers and oligomers including those synthesized from materials such as lactic acid, glycolic acid, caprolactone, dioxanone, polyethylene glycol (PEG), polypropylene glycol, isocyanates, copolymers thereof, combinations thereof, and the like. Adhesive materials may be combined with solvents for their application, including polar and non-polar solvents. Suitable solvents include alcohols, e.g., methanol, ethanol, propanol, chlorinated hydrocarbons (such as methylene chloride, chloroform, 1,2-dichloro-ethane), and aliphatic hydrocarbons such as hexane, heptene, ethyl acetate.

The specific manner in which the binding agent may be applied to the yarn 10 will depend on the contemplated uses of the suture. After the binding agent is applied to the strands 12 and 14, strands 12 and 14 and/or the binding agent may be set using known curing methods or processes (e.g., ultra-violet curing) or other suitable methods or processes not described herein.

In embodiments, the strands 12 and 14 and/or the binding agent may be simultaneously subjected to an ultra-violet curing process. Following the ultra-violet curing process, the forming sheath may be subsequently removed, producing a yarn 10 that includes the strands 12 and 14 in a fixed parallel configuration.

In embodiments, after the strands 12 and 14 have been set or formed in the fixed parallel configuration, the yarn 10 including the strands 12 and 14 may be treated with a suitable coating (not shown). In embodiments, the coating may be employed to facilitate in maintaining the strands 12 and 14 in a parallel configuration. Any biocompatible coating may be applied thereto, including those disclosed in U.S. Patent Application Publication Nos. 2008/0268243, 2007/0207189, 2007/0010856, 2006/0188545, 2004/0153125, and 2004/0147629, and U.S. Patent Nos. 6,878,757, 6,136,018, 6,007,565, and 5,716,376, the entire disclosures of each of which are incorporated by reference herein.

A coating and/or sheath may be applied in discrete locations or, in embodiments, may be applied along the longitudinal distance of the suture. Where applied in discrete locations, the locations may be intermittent and/or discrete, or may be along one or more partial lengths of continuous and/or increasing and/or decreasing change in length along the suture.

Once formed, a plurality of the heterogeneous yarns 10 can then be braided, knitted, or woven together. The braiding can be done by any method known to those skilled in the art. For example, braid constructions for sutures and other medical devices are described in U.S. Pat. Nos. 5,019,093; 5,059,213; 5,133,738; 5,181,923; 5,226,912; 5,261,886; 5,306,289; 5,318,575; 5,370,031; 5,383,387; 5,662,682; 5,667,528; 6,203,564; the contents of each of which are incorporated by reference herein. Once the suture is constructed, it is preferably sterilized, by any means known to those skilled in the art.

Braided surgical devices made using heterogeneous yarns prepared in accordance with the disclosure can optionally be coated with one or more coating compositions to improve functional properties of the device. For example, a coating can be applied to improve surface lubricity and knot tie-down behavior. Suitable coating compositions include but are not limited to those disclosed in U.S. Pat. Nos. 3,867,190; 3,942,532; 4,047,533; 4,452,973; 4,624,256; 4,649,920; 4,716,203; 4,826,945; and 5,569,302, the disclosures of which are incorporated by reference herein. The coating can be applied using any known technique such as, for example coating, dipping, spraying or other appropriate techniques.

The amount of coating composition applied to the device will vary depending upon the specific construction of the device, its size and the exact material of its construction. In general, the coating composition will constitute from about 0.5 to about 4.0 percent by weight of the coated device or higher with a preferred range from about 1.0 percent to about 3.0 percent.

A surgical device prepared from the presently described heterogeneous yarns may also be impregnated with one or more medico-surgically useful substances, e.g., those which accelerate or beneficially modify the healing process when the suture is applied to a wound or surgical site. The medically useful or therapeutic agents can include varying amounts of one or more optional ingredients, such as, for example, bioactive substances such as biocidal agents, antibiotics, antimicrobials, medicants, growth factors, anti-clotting agents, analgesic, anesthetics, anti-inflammatory, etc., and the like. Medicants are defined as substances which are beneficial to the animal and tend to promote the healing process. For example, a braided suture can be provided with a therapeutic agent which will be deposited at the sutured site. The therapeutic agent can be chosen for its antimicrobial properties, capability for promoting wound repair and/or tissue growth, or for specific indications such as thrombosis. Antimicrobial agents such as broad spectrum antibiotics (gentamicin sulphate, erythromycin or derivatized glycopeptides) which are slowly released into the tissue can be applied in this manner to aid in combating clinical and sub-clinical infections in a surgical or trauma wound site. To promote wound repair and/or tissue growth, one or more biologically active materials known to achieve either or both of these objectives can also be applied to the braided suture. Such materials include any of several human Growth factors (HGFs), magainin, tissue or kidney plasminogen activator to cause thrombosis, superoxide dismutase to scavenge tissue-damaging free radicals, tumor necrosis factor for cancer therapy, colony stimulating factor, interferon, interleukin-2 or other lymphokines to enhance the immune system, and so forth.

The braided device prepared from heterogeneous yarns in accordance with this disclosure can also include, for example, biologically acceptable plasticizers, antioxidants, and colorants, which can be impregnated into the heterogeneous yarns of the device.

In addition, the yarn and/or product may be plasma treated depending upon the particular needs or intended application so as to reduce the perceived "slipperiness" of the product as desired.

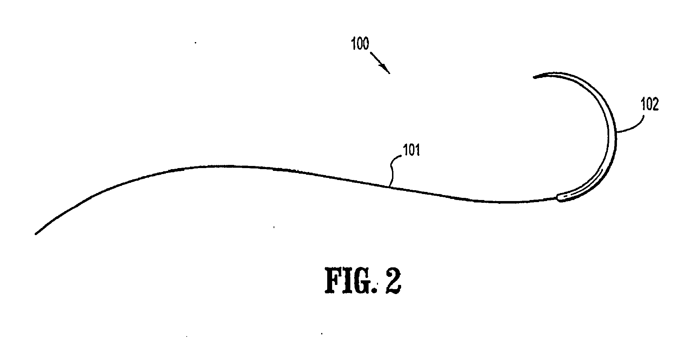

The braided suture 101 prepared in accordance with this disclosure can have a needle 102 attached thereto as shown in FIG. 2, to provide a needle suture combination 100. In order to facilitate needle attachment, conventional tipping agents can be applied to the braid. Two tipped ends of the suture may be desirable for attaching a needle to each end of the suture to provide a so-called double armed suture. The needle attachment can be made by any conventional method such as crimping, swaging, etc., including those described in U.S. Pat. Nos. 5,133,738; 5,226,912; and 5,569,302, the disclosures of which are incorporated by reference herein.

As noted above the heterogeneous yarns of the present disclosure can also be utilized to form other surgical or medical articles, including, braided tapes, gauze, wound dressings, hernial repair meshes, vascular grafts (e.g. fabrics and/or tubes) anastomosis rings, prosthetic ligaments and tendons, growth matrices, drug delivery devices and other implantable medical devices.

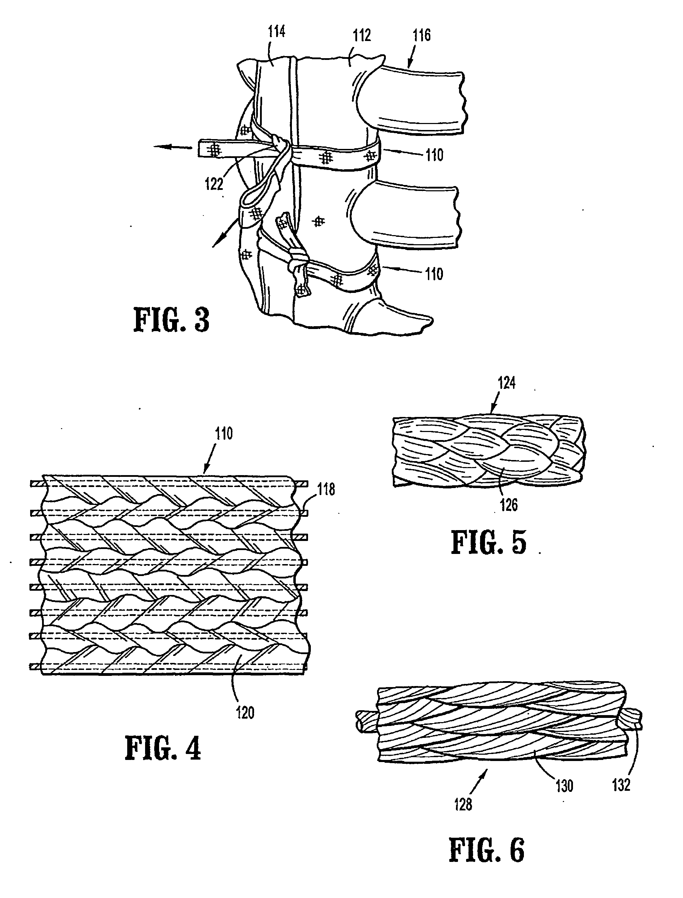

Referring initially to FIG. 3 there is illustrated a sternum closure ribbon 110 constructed according to the present invention and positioned to retain portions 112, 114 of a human sternum 116 together. The band 110 is a braided product made from the heterogeneous yarns described herein. In FIG. 4, the band 110 shown in FIG. 3 is shown in greater detail as an elongated flat braided textile product prepared as described in U.S. Pat. No. 5,318,575, the disclosure of which is incorporated herein by reference, with the exception that heterogeneous yarns in accordance with the present disclosure are used in the construction.

Accordingly, it is possible in one application to position the reinforced structure 110 about the split portions 112, 114 of the human sternum 116 as shown in FIG. 3 whereby substantial force may be applied to the band by tying the band either by a knot 122 shown in FIG. 3, or by other techniques whereby significant force may be applied and retained to promote natural healing of the sternum portions 112, 114, e.g. mechanical connecting devices such as buckles, etc. See, for example, U.S. Pat. No. 4,813,416.

In FIG. 5, there is an alternative elongated embodiment of spiroid braided construction of generally circular cross-section and comprised of heterogeneous yarns 126 combined to form a braided rope-like construction of generally circular cross-sectional configuration. Braid constructions having a circular cross-section are described in U.S. Pat. Nos. 3,565,077 and 5,019,093. In FIG. 6 there is shown a hollow braid construction 128 having a sheath constructed of heterogeneous yarns 130 and having a core 132.

Surgical devices prepared from heterogeneous yarns in accordance with this disclosure can be packaged and sterilized in any conventional manner known to those skilled in the art.

It will be understood that various modifications may be made to the embodiments disclosed herein. For example, in any of the braided products described herein one or more of the yarns may be heterogeneous yarns in accordance with this disclosure while the remaining portions are made of absorbable or non-absorbable fibers or filaments. As one illustrative example, for braided products containing a core/sheath structure, the core may be a heterogeneous yarns in accordance with this disclosure while the sheath yarns can be made form other biocompatible fibers, including, but not necessarily, bioabsorbable fibers. Therefore the above description should not be construed as limiting, but merely as exemplications of preferred embodiments. Those skilled in the art will envision other modifications within the scope and spirit of the claims appended hereto.

## Claims

1. A surgical device comprising: at least one heterogeneous yarn defining a longitudinal axis and including a plurality of linear disposed polyester strands in intimate contact with a plurality of linear disposed polyolefin strands, wherein the plurality of polyester strands and the plurality of the polyolefin strands run parallel to each other and the longitudinal axis along the length of the yarn.

2. A surgical device as in claim 1, wherein the polyester strands comprise polyethylene terephthalate and/or a surgical device as in claim 1, wherein the polyolefin strands comprise polyethylene, preferably wherein the polyolefin strands comprise ultra high molecular weight polyethylene.

3. A surgical device as in claim 1 or claim 2, wherein the at least one heterogeneous yarn further includes strands made from a material other than polyolefin or polyester.

4. A surgical device as in any of claims 1 to 3, comprising a plurality of heterogeneous yarns that are braided; and/or a surgical device as in any of claims 1 to 3, comprising a plurality of heterogeneous yarns that are knitted; and/or a surgical device as in any of claims 1 to 3, comprising a plurality of heterogeneous yarns that are woven.

5. A biocompatible surgical device comprising a textile prepared from a plurality of heterogeneous yarns each defining a longitudinal axis and having linear disposed strands of polyethylene terephthalate in intimate contact with linear disposed strands of ultra high molecular weight polyethylene, wherein the plurality of polyethylene terephthalate strands and the plurality of the ultra high molecular weight polyethylene strands run parallel to each other and the longitudinal axis along the length of the yarn.

6. A biocompatible surgical device as in claim 5, wherein the textile comprises braided heterogeneous yarns; and/or a biocompatible surgical device as in claim 5, wherein the textile comprises knitted heterogeneous yarns; and/or a biocompatible surgical device as in claim 5, wherein the textile comprises woven heterogeneous yarns.

7. A biocompatible surgical device as in claim 5 or claim 6, wherein the textile is adapted for use as a device selecting from the group consisting of sutures, tapes, gauze, wound dressings, hernial repair meshes, vascular grafts, anastomosis rings, prosthetic ligaments, prosthetic tendons, growth matrices and drug delivery devices.

8. A biocompatible surgical device as in any of claims 5 to 7, wherein the textile is a braided suture.

9. A suture comprising at least one heterogeneous yarn defining a longitudinal axis and having linear disposed polyester strands in intimate contact with linear disposed polyolefin strands, wherein the plurality of polyester strands and the plurality of polyolefin strands run parallel to each other and the longitudinal axis along the length of the yarn.

10. A suture as in claim 9, wherein the polyester strands comprise polyethylene terephthalate.

11. A suture as in claim 9 or claim 10, wherein the polyolefin strands comprise polyethylene.

12. A suture as in claim 11, wherein the polyolefin strands comprise ultra high molecular weight polyethylene.

13. A suture as in any of claims 9 to 12, wherein the heterogeneous yarn is coated with a composition containing one or more biologically active materials.

14. A surgical device as in any preceding claim, wherein the plurality of polyester strands and polyolefin strands are grouped together forming a plurality of interstices that run parallel to each other and the polyester and polyolefin strands along the length of the yarn.

15. A method of manufacturing a yarn for use with a surgical device comprising:
positioning a plurality of heterogeneous stands in side-by-side fashion such that the plurality of heterogeneous strands are maintained in a parallel relation to one another;
applying a binding agent to the plurality of heterogeneous strands; and setting the binding agent.
